# EUROPEAN PATENT APPLICATION

(11) **EP 3 895 720 A1**
(43) Date of publication of application: **20.10.2021**
(21) Application number: 20382301.8
(22) Date of filing: 15.04.2020
(51) Int. Cl.: A61K 36/185, A61K 47/00

(54) **METHOD FOR OBTAINING A BOTANICAL EXTRACT**

(71) Applicant: Euromed, S.A., 08100 Mollet Del Vallès (Barcelona) (ES)
(72) Inventor: ROIG ALMIRALL, Francisco Javier, 08021 Barcelona (ES); VILLAR GONZALEZ, Agustín, 08291 Ripollet (Barcelona) (ES); GUERRERO MARTINEZ, Juan Ramón, 30152 Aljucer (Murcia) (ES); VERGES BENET, Juan Antonio, 08100 Mollet de Vallès (Barcelona) (ES)
(74) Representative: Durán-Corretjer, S.L.P.

(57) **Abstract**

The present invention discloses a method for preparing a botanical extract characterised in that it comprises the steps of: (a) extracting plant matter with a solvent, and separating the solid plant matter from the liquid fraction; (b) concentrating the liquid fraction; (c) purifying the liquid fraction obtained in step b) by tangential flow filtration.

## Description

The present invention relates to a method for preparing various botanical extracts enriched with active substances from plant raw materials for use in the cosmetic, chemical, pharmaceutical or food industry, among others, in which a step of tangential flow filtration (TFF) is used.

Botanical extracts have a wide range of properties, for example as digestive stimulants, anti-diarrhoeals, antiseptics, anti-inflammatories, appetite stimulants, gastric stimulants, among many other properties.

For the pharmaceutical industry, botanical extracts are a source of new molecules with pharmacological effects, which can be used directly and which make it possible to obtain pharmaceutical products with fewer side effects and to satisfy the growing needs for the use of natural products.

One of the most important aspects in the production of botanical extracts is to guarantee high yields from the plant matter and, therefore, a high content of active substances. This depends, among other aspects, on the appropriate choice of plant matter, the availability of the species, the feasibility of cultivation, the place and time of cultivation and botanical identification, as well as the selection, drying, milling and storage of the botanical extract.

Nowadays, botanical extracts are usually obtained by a solid-liquid extraction in which the methods of extraction by maceration and percolation or leaching are the most used.

For the extraction of such botanical extracts, it is important to establish the extraction parameters in order to achieve the standardisation of the process, i.e. to select the type of solvent, the solid-liquid ratio, the particle size of the solid, the smaller the particle size, the larger the contact surface between the plant matter and the solvent, the temperature, the stirring speed and extraction time, the viscosity of the medium, among others. This will ensure the quality, yield, safety and efficacy of the medicinal product.

The plant extract obtained must be characterised in terms of its active substances and markers, density, residual solvents, total solids, pH, microbiological control and content of impurities such as pesticides, heavy metals or other potentially undesirable compounds such as alkaloids, aflatoxins, among other parameters.

Taking into account the botanical extract to be obtained, one type of extraction method or another among those known in the prior art should be used. The botanical extract to be extracted is different according to the desired active substance, so for each extraction the type of method and its parameters must be adapted, which makes it difficult to standardise a single process for any type of botanical extract. Therefore, it is necessary to develop processes that can be used for a wide variety of botanical extracts without the need to modify the parameters and characteristics of the method for each of the botanical extracts.

The inventors of the present invention have discovered, surprisingly, that with the use of a method that comprises a step of extraction with a solvent and a step of tangential flow filtration, it is possible to obtain a wide variety of botanical extracts with suitable quality characteristics, without the need to use different parameters in each of the botanical extracts. Therefore, the combination of a step of extraction with a solvent and a step of purification by tangential flow filtration allows the enrichment of some of the active substances of the botanical extract, maintaining the medicinal and/or cosmetic properties of said extract.

Tangential flow filtration is a very versatile technique that allows for everything from removing suspended solids to purifying compounds, depending on the membrane selected. In this technique, the recirculation of the product under pressure avoids the clogging of the filters, allowing filtrations that, with another type of filter, would be non-viable. Using the appropriate membranes, tangential filtration can also purify extracts. The TFF technique consists of a pressurised recirculation current and a permeated liquid flow that passes through the membranes. As indicated above, with the choice of the appropriate membrane, the substance of interest (polyphenols or other molecules of the extract obtained) may or may not be able to pass through the membrane. In both cases, the compound will undergo a process of purification to a greater or lesser extent. This technique ensures stable yields of the desired filtration level.

In the present invention this tangential flow filtration can be used even in methods that use organic solvents, with membranes suitable for them, as well as aqueous solvents and even water.

In addition, the use of tangential flow filtration means that the botanical extract does not need to undergo chemical or physical processes (such as heating processes) for the purification thereof and that might reduce the quality thereof due to the degradation of the possible thermolabile substances that make it up.

The improvement of the extraction process makes it possible to overcome the barriers of industrialisation caused by the difficulty in scaling up the processes in the case of raw materials of plant origin, which makes it possible to work with a large amount of raw materials and to obtain quantities of product enriched with active substances with very high purity. This difficulty can be summarised in the concept of the raw material to final extract ratio, known as the *drug-to-extract ratio* (DER). In cases where this purification technique is applied, the DER values are between 4 and 1, and even between 200 and 1 or more, and can be up to 1000 to 1.

In the present invention the term "plant raw material" refers to anything that can be used or consumed directly from nature and that is processed to produce plant extracts that will later be converted into consumer goods. Plant matter can be obtained from the root, leaves, stem, fruit, seeds, inflorescence, among other parts of the plants.

In a first aspect, the present invention discloses a method for preparing a botanical extract characterised in that it comprises the steps of: (a) extracting plant matter with a solvent, and separating the solid plant matter from the liquid fraction; (b) concentrating the liquid fraction; (c) purifying the liquid fraction obtained in step b) by tangential flow filtration.

Preferably, said solvent in the extraction step is an organic solvent or an aqueous solution of such an organic solvent. More preferably, said solvent is ultrapure water.

Ultrapure water is made up exclusively of water molecules with hydrogen and hydroxyl ions in equilibrium (10⁻⁷ M at 25ºC) which gives it a characteristic electrical resistivity of 18.2 MOhm.cm. Furthermore, ultrapure water is 99.999999975% pure and lacks all non-gaseous and inorganic elements. For example, one type of ultrapure water is that obtained by several consecutive steps of reverse osmosis.

Preferably, the plant matter is selected from the group formed by pomegranate, cucumber, broccoli, strawberry, *Epilobium angustifolium,* blueberry, grape or red vine, chamomile, maypop, parsley, sage, arnica, mint, citrus, orange, lemon, grapefruit, cinnamon, *Serenoa repens,* nettle, sunflower, spinach, olive leaf, magnolia, bearberry, grape seed, olive, cannabis, coffee beans, artichoke flower, yerba mate, cocoa, *Thea sinensis,* ginseng, horse chestnut, turmeric, pine bark, artichoke plant, echinacea, ginseng, persimmon, pear, among others.

Preferably, the final extract obtained contains the active substances selected from the group formed by alkaloids, ellagitannins, anthocyanins, flavones and flavonols, flavonoids, sterols, ecdysteroids, steroids, iridoids, lignans, phenols, curcuminoids, cannabinoids, phenylpropanoids, saponins, oligomeric procyanidins, imino and amino sugars, among others. Most preferably, said active substance is not abscisic acid.

The plant matter can undergo extraction in fresh state or, with the aim of making it viable to preserve it, in dried state. Preferably, the plant matter is dried. Preferably, the water content of said plant matter does not exceed 10% by weight.

In the extraction method, said plant matter can be used whole, or mechanically crushed, as a step prior to extraction. In this case, any of the methods available to the person skilled in the art for crushing or cutting the plant matter are suitable.

In addition, the plant matter used for extraction can be the result of prior industrial processing, such as by-products of the food industry, among others.

Preferably, a volume of solvent equivalent to 3 to 6 times the weight of the plant matter to be extracted is used in the extraction step.

In a preferred embodiment, two consecutive extractions are performed.

In a preferred embodiment, the extraction is carried out at a temperature between 50ºC and 75ºC.

In a preferred embodiment, the membrane size in the tangential flow filtration is between 100 Da and 100,000 Da.

In a preferred embodiment, the pressure in the tangential flow filtration is between 4 and 30 bar.

In a preferred embodiment, the pH in the tangential flow filtration step is between 2 and 9. More preferably, the pH in the tangential flow filtration step is between 2 and 5.

Agents that improve the fluidity of the liquids to be passed through the filtration membrane can also be used. These agents must be soluble in the solvent of the solution to be filtered and can be of different natures such as surfactants or surface-active agents, thickeners, emulsifiers, among others. Among these it may be preferable to use glycerine or propylene glycol if an agent is needed for this purpose. In a preferred embodiment, glycerine is used in the tangential flow filtration step.

Furthermore, the present invention discloses a composition comprising active substances selected from the group formed by alkaloids, ellagitannins, anthocyanins, flavones and flavonols, flavonoids, sterols, ecdysteroids, steroids, iridoids, lignans, phenols, curcuminoids, cannabinoids, phenylpropanoids, saponins, oligomeric procyanidins, imino and amino sugars, among others. Most preferably, said active substance is not abscisic acid.

Preferably, said composition comprises at least one additional component. Preferably, the additional component is selected from the group formed by colloidal silica, talc, tricalcium phosphate, magnesium stearate, carbohydrates, polysaccharides, maltodextrins, water, polyols, celluloses, starches, and mixtures thereof. Preferably, the additional component is present between 0.5% (w/w) and 35% (w/w). More preferably, approximately 20% (w/w) of maltodextrin. Even more preferably, approximately 2% (w/w) of silicon dioxide or tricalcium phosphate.

With the method of the present invention it is possible to obtain an active substance content that is between 3 and 18 times higher than the initial active substance content.

Several examples are provided below to illustrate, but not limit, the invention.

### Examples

### EXAMPLE 1: Preparation of 10 kg of crushed cucumbers mixed with 30 kg of purified water.

The mixture was exposed to a temperature of 70ºC using two extraction steps. The plant matter was separated from the micelle by solid-liquid filtration resulting in a liquid with a solids concentration of 6%. Said micelle underwent a tangential filtration process using a filter cartridge with a nanofiltration membrane (300 Da). The working pressure during the filtration process was between 4-30 bar.

The result of the filtration was that 80% of the active substance (total amino derivatives, including imino and amino sugars) was recovered in the concentrate phase. The purity of the active substance in the final product was 12% in the permeate phase which meant a purification by 3 times of the initial content.

### EXAMPLE 2: Preparation of 10 kg of crushed cucumbers mixed with 30 kg of purified water.

The mixture was exposed to a temperature of 70ºC using two extraction steps. The plant matter was separated from the micelle by solid-liquid filtration resulting in a liquid with a solids concentration of 6%.

Said micelle underwent a tangential filtration process using a filter cartridge with a nanofiltration membrane (300 Da). For this process the pH of the micelle was adjusted to a value between 2-5 by the addition of a mineral acid. The working pressure during the filtration process was between 4-30 bar.

The result of the filtration was that 80% of the active substance (total amino derivatives) was recovered in the concentrate phase. The purity of the active substance in the final product was 17% in the permeate phase which meant a purification by 4 times of the initial content.

In this case the pH adjustment achieved an unexpected and not initially obvious purification effect in the extract that was filtered through the membrane.

### EXAMPLE 3: Preparation of 10 kg of crushed cucumbers mixed with 30 kg of purified water.

The mixture was exposed to a temperature of 50°C and the plant matter was separated from the micelle by solid-liquid filtration resulting in a liquid with a solids concentration of 6%. Said micelle underwent a tangential filtration process using a filter cartridge with a nanofiltration membrane (300 Da). For this process the pH of the micelle was adjusted to a value between 2-5 by the addition of a mineral acid.

To aid filtration, 2% of glycerine was added to the mixture and the working pressure during the filtration process was between 4-30 bar.

The result of the filtration was that 83% of the active substance (total amino derivatives) was recovered in the concentrate phase. The purity of the active substance in the final product was 25% in the permeate phase which meant a purification by 6 times of the initial content.

In this case the pH adjustment and the addition of glycerine as an additive achieved an unexpected and not initially obvious purification effect in the extract that was filtered through the membrane.

### EXAMPLE 4: Extraction of 5 kg of magnolia bark by a mixture of 60% alcohol and water at 55ºC using two extraction steps.

The plant matter was separated from the micelle by solid-liquid filtration resulting in a liquid with a solids concentration of 4.5% and a flavonolignan content of 2.5%. Said micelle underwent a tangential filtration process using a filter cartridge with a large-pore membrane (1000- 10,000 Da) suitable for the passage of organic solvents. For this process the pH of the micelle was adjusted to a value between 5-9 by adding a mineral base. The working pressure during the filtration process was between 4-30 bar.

The result of the filtration was that in the permeate phase 65% of the initial flavonolignans were recovered which meant a purification by 3 times of the initial content.

### EXAMPLE 5: Extraction of 8 kg of crushed turmeric root with 30 kg of alcohol diluted with water to 85%.

The plant matter was separated from the micelle by solid-liquid filtration resulting in a liquid with a solids concentration of 3.0% and a curcuminoid content of 17%. Said micelle underwent a tangential filtration process using a filter cartridge with a nanofiltration membrane capable of filtering substances dissolved in organic solvents (300 Da). For this process the pH of the micelle was adjusted to a value between 7 and 8.5 by adding an alkaline substance (sodium bicarbonate). The working pressure during the filtration process was between 4-30 bar.

The result of the filtration was that 76% of the total initial curcuminoids were recovered in the permeate phase. The content in the final product was increased to values of 45% which meant a purification by 3 times of the initial content without the need to use organic solvents other than ethanol.

### EXAMPLE 6: Extraction of 4 kg of cocoa powder with purified water.

The solid was separated from the liquid by filtration and the resulting liquid was concentrated by evaporation to a dry residue of more than 75%. The concentrate was mixed with ethanol in a ratio of 1 to 10. After stirring, the result was a precipitation of the sugars present in the solution. This solution was then filtered and the precipitate was discarded.

The liquid was adjusted to a dry residue of 2% with ethanol and passed through nanofiltration membranes capable of working with organic solvents *(Organic solvent nanofiltration membranes,* OSN). Specifically, a 900 Da membrane was used in diafiltration mode.

The result of the described process makes it possible to obtain a cocoa extract with a high content of flavan-3-ols and a reduction of the alkaloid content.
There are conventional methods for performing this reduction of alkaloids in cocoa, which can be contaminating since they use highly hazardous organic solvents such as chlorine products or solvents that can interfere with the aim of preserving the flavan-3-ols in the final product, such as ethyl acetate that would remove the alkaloids and also the flavan-3-ols. The same would happen with water.

This method is an efficient, environmentally friendly, reproducible way that also preserves the properties of the product.

### EXAMPLE 7: Increase of the flavan-3-ol content in a sample of grape seed extract.

To extract the grape seeds, ultrapure water was used as a solvent. The solid was separated from the liquid by filtration and the resulting liquid was concentrated by evaporation to a dry residue of more than 75%. The concentrate was mixed with ethanol in a ratio of 1 to 10. After stirring, the result was a precipitation of the sugars present in the solution. This solution was then filtered and the precipitate was discarded.

The liquid was adjusted to a dry residue of 2% with ethanol and passed through nanofiltration membranes capable of working with organic solvents *(Organic solvent nanofiltration membranes,* OSN). Specifically, a 900 Da membrane was used in diafiltration mode.

The result of this operation was an increase of more than three times in the flavan-3-ol content of the extract.

### EXAMPLE 8: Increase of the flavan-3-ol and total polyphenol content in a sample of pine bark extract.

To extract pine bark, ultrapure water was used as a solvent. The solid was separated from the liquid by filtration and the resulting liquid was concentrated by evaporation to a dry residue of more than 75%. The concentrate was mixed with ethanol in a ratio of 1 to 10. After stirring, the result was a precipitation of the sugars present in the solution. This solution was then filtered and the precipitate was discarded.

The liquid was adjusted to a dry residue of 2% with ethanol and passed through nanofiltration membranes capable of working with organic solvents *(Organic solvent nanofiltration membranes,* OSN). Specifically, a 900 Da membrane was used in diafiltration mode.

The result of this operation was an increase of more than three times in the flavan-3-ol and total polyphenol content of the extract.

EXAMPLE 9: Extraction of 10 kg of spinach selected so as to obtain a product with high ecdysteroid content.

The extraction was carried out with water at 65ºC and after that the solid was separated from the liquid by filtration.

A micelle was obtained with a dry residue of around 3% and a total of 3 kg of dry matter. The ecdysteroid content of the product was 0.19% of the dry matter.

Said micelle underwent a tangential filtration process using a filter cartridge with a nanofiltration membrane (300 Da). For this process the pH of the micelle was adjusted to a value between 3-5 by the addition of a mineral acid. The working pressure during the filtration process was between 4-30 bar.

The result of the filtration was that 95% of the initial ecdysteroids were recovered in the concentrate phase compared to the 10% obtained in the permeate phase. This meant an effective purification of around 4 times the ecdysteroid content.

### EXAMPLE 10: Extraction of 10 kg of lemon rind with water at 50°C for 4 hours.

The liquid was separated from the undissolved solid by centrifugation. The extract contained the compound of interest, eriocitrin, a flavonoid that is abundant in citrus fruits.

The micelle with dry residue of 5% underwent a process of purification by tangential cascade filtration. For these, three linked steps were carried out:
1. The micelle was filtered through a 100,000 Da membrane, with 99% of the eriocitrin passing into the permeate and obtaining 3% of dry residue.
2. The permeate of the first step was filtered through a 10,000 Da membrane, 99% of the compound of interest passing into the permeate and obtaining 1% of solids.
3. The permeate of step 2 was filtered through a 1000 Da membrane, 98% of the eriocitrin passing into the permeate and obtaining 0.5% of solids.

In the three steps as a whole, the liquid extract went from 5% of solids to 0.5% of solids, while maintaining the same amount of eriocitrin. Therefore, it was purified 10 times.

### EXAMPLE 11: Extraction of 10 kg of lemon rind with water at 50°C for 4 hours.

The liquid was separated from the undissolved solid by centrifugation. The extract contained the compound of interest, eriocitrin.

The micelle with dry residue of 5% underwent a process of purification by tangential cascade filtration. For these, three linked steps were carried out:
1. The micelle was filtered through a 100,000 Da membrane, with 99% of the eriocitrin passing into the permeate and obtaining 3% of dry residue.
2. The permeate of the first step was adjusted to a basic pH by adding an alkaline substance (sodium hydroxide) and the solution was filtered through a 10,000 Da membrane, 99% of the compound of interest passing into the permeate. The pH caused a higher retention of solids which finally resulted in 0.5% of solids in the permeate.
3. The permeate of step 2 was filtered through a 1000 Da membrane, 98% of the eriocitrin passing into the permeate and obtaining 0.25% of solids.

In the three steps as a whole, the liquid extract went from 5% of solids to 0.25% of solids, while maintaining the same amount of eriocitrin. Therefore, it was purified a total of 18 times.

## Claims

1. A method for preparing a botanical extract **characterised in that** it comprises the steps of:
a) extracting a plant matter with a solvent, and separating the solid plant matter from the liquid fraction,
b) concentrating the liquid fraction,
c) purifying the liquid fraction obtained in step b) by tangential flow filtration.

2. The method according to claim 1, **characterised in that** said solvent is an organic solvent or an aqueous solution of said organic solvent.

3. The method according to claim 1, **characterised in that** said solvent is ultrapure water.

4. The method according to any preceding claim, **characterised in that** the plant matter is selected from the group formed by pomegranate, cucumber, broccoli, strawberry, *Epilobium angustifolium,* blueberry, grape or red vine, camomile, maypop, parsley, sage, arnica, mint, citrus, orange, lemon, grapefruit, cinnamon, *Serenoa repens,* nettle, sunflower, spinach, olive leaf, magnolia, bearberry, grape seed, olive, cannabis, coffee beans, artichoke flower, yerba mate, cocoa, *Thea sinensis,* ginseng, horse chestnut, turmeric, pine bark, artichoke plant, echinacea, ginseng, persimmon, pear.

5. The method according to any preceding claim, **characterised in that** the extract obtained contains the active substances selected from the group formed by alkaloids, ellagitannins, anthocyanins, flavones and flavonols, flavonoids, sterols, ecdysteroids, steroids, iridoids, lignans, phenols, curcuminoids, cannabinoids, phenylpropanoids, saponins, oligomeric procyanidins, imino and amino sugars.

6. The method according to any preceding claim, **characterised in that** said active substance is not abscisic acid.

7. The method according to any one of claims 1 to 4, **characterised in that** the plant matter is dried.

8. The method according to any preceding claim, **characterised in that** a volume of solvent equivalent to between 3 and 6 times the weight of the plant matter to be extracted is used.

9. The method according to any preceding claim, **characterised in that** the extraction is carried out at a temperature of 50°C to 75ºC.

10. The method according to any preceding claim, **characterised in that** the size of the membrane in the tangential flow filtration is between 100 Da and 100,000 Da.

11. The method according to any preceding claim, **characterised in that** the pressure in the tangential flow filtration is between 4 and 30 bar.

12. A composition **characterised in that** it comprises the botanical extract obtained according to claims 1 to 11 and at least one additional component.

13. The composition according to claim 12, **characterised in that** the extract obtained contains the active substances selected from the group formed by alkaloids, ellagitannins, anthocyanins, flavones and flavonols, flavonoids, sterols, ecdysteroids, steroids, iridoids, lignans, phenols, curcuminoids, cannabinoids, phenylpropanoids, saponins, oligomeric procyanidins, imino and amino sugars.

14. The composition according to claims 12 and 13, **characterised in that** the additional component is selected from the group formed by colloidal silica, talc, tricalcium phosphate, magnesium stearate, carbohydrates, polysaccharides, maltodextrins, water, polyols, cellulose, starches, and mixtures thereof.

15. The composition according to claim 14, **characterised in that** it comprises 0.5% (w/w) to 35% (w/w) of an additional component.

16. The composition according to claim 15, **characterised in that** it comprises approximately 20% (w/w) of maltodextrin.

17. The composition according to claim 16, **characterised in that** it comprises approximately 2% (w/w) of silicon dioxide or tricalcium phosphate.
